# FASCICULE DE BREVET EUROPEEN

(11) **EP 1 566 759 B1**
(45) Date de publication et mention de la délivrance du brevet: **07.04.2010**
(21) Numéro de dépôt: 04300916.6
(22) Date de dépôt: 17.12.2004
(51) Int. Cl.: G06F 19/00

(54) **Dispositif de saisie, de consultation et de traitement de données, notamment médicales**
Vorrichtung zum Eingeben, Betrachten und Verarbeiten von Daten, insbesondere medizinischen Daten
Device for inputting, consulting and processing of data, in particular medical data

(30) Priorité: 02.02.2004 FR 0450187
(43) Date de publication de la demande: 24.08.2005
(73) Titulaire: MEDISCS SAS, 34470 Perols (FR)
(72) Inventeur: Rabischong, Pierre, 34000, MONTPELLIER (FR); Arnail, Yves, 12370, BELMONT SUR RANCE (FR); Delbourg, Bernard, 26, Allée M. Geraud, 34000, MONTPELLIER (FR)
(74) Mandataire: Rhein, Alain

(56) Documents cités:
- WO-A-03/046827
- US-A1- 2002 123 909
- US-A1- 2003 040 940
- US-A1- 2003 056 204
- US-A1- 2003 088 439

## Description

La présente invention concerne un dispositif de saisie, de consultation et de traitement de données, notamment médicales, comprenant au moins un support informatique mobile pour l'enregistrement de données, un lecteur-graveur dudit support mobile, au moins un support informatique fixe pour l'enregistrement de données, ledit support informatique fixe et ledit support informatique mobile étant aptes à communiquer entre eux à distance, via un réseau de communication, de type Internet par exemple, au travers dudit lecteur-graveur.

L'objectif d'une telle invention est principalement d'offrir un support informatique mobile, par exemple sous la forme d'un CD-ROM (Compact Disc - Read Only Memory) ou d'un DVD (Digital Versatile Disc), permettant de stocker le dossier médical d'un patient de manière que ce dernier puisse y accéder par lui-même, comme la loi l'y autorise, à partir d'un lecteur approprié.

Un autre objet de l'invention consiste à proposer aux médecins un système leur permettant de compléter plus facilement les dossiers médicaux de leurs patients et leur offrant, le cas échéant, une assistance pour établir un diagnostic.

L'on connaît du document WO 03/046827 une méthode et un dispositif pour stocker des données relatives à un individu et accéder à ces données de manière sécurisée, qui trouvent une application dans le domaine médical. Le dispositif objet de cette demande de brevet comprend un moyen de stockage de données portable (par exemple un CD-ROM) comportant un registre de données cryptées et des moyens de décryptage aptes à décrypter lesdites données suite à la fourniture d'une clé (mot de passe) par l'utilisateur, un ordinateur, avec lequel le moyen de stockage de données portable peut être couplé, ledit ordinateur étant équipé de moyens autorisant l'accès aux données stockées sur le moyen de stockage, des moyens aptes à déclencher les moyens de décryptage et des moyens d'affichage des données décryptées.

En fait, le dispositif objet de ce document est prévu apte à permettre la lecture des données personnelles d'un utilisateur stockées sur un CD-ROM, à partir d'un ordinateur équipé d'un lecteur de CD-ROM, la sécurisation des données étant assurée par leur cryptage et la nécessité de disposer d'un mot de passe provoquant leur décryptage.

Le stockage des données sur le CD-ROM s'effectue au travers d'une installation distante comprenant un "serveur" relié à des moyens de création de CD-ROM. Ainsi, pour créer un moyen de stockage de données portable (CD-ROM), l'utilisateur entre ses données personnelles dans un ordinateur "client" relié au "serveur" au travers d'une liaison Internet. Les données sont envoyées au "serveur" puis vers les moyens de création de CD-ROM par le biais d'une liaison sécurisée. Les données sont ensuite transformées en pages Web, conjointement à la création d'une page Web contenant le mot de passe permettant à l'utilisateur d'accéder à ses données, puis gravées sur un CD-ROM ou, le cas échéant, transmises vers une base de données.

Ainsi, le dispositif objet du document WO 03/046827 permet à un utilisateur de disposer d'un CD-ROM comportant des données personnelles, puis d'accéder à ces dernières pour les consulter, une fois le CD-ROM créé, depuis n'importe quel ordinateur équipé d'un lecteur de CD-ROM.

Par contre, le dispositif n'offre pas la possibilité à l'utilisateur ou à une personne habilitée par l'utilisateur, de modifier directement les données stockées sur le CD-ROM, depuis n'importe quel ordinateur. Il implique à cet effet l'envoi d'éventuelles données complémentaires ou rectificatives d'un ordinateur client vers le serveur puis la base de données distante sur laquelle elles sont finalement détenues sous forme cryptée ou non. Leur consultation s'effectue ensuite au travers du CD-ROM, disposant des moyens permettant d'accéder à la base de données.

Ainsi, le document WO 03/046827 concerne, entre autres, un dispositif de stockage de données relatives à un individu, et d'accès à ces données, faisant intervenir un moyen de stockage de données portable et une base de donnée hébergée par un serveur, dans lequel le moyen de stockage de données portable comprend des moyens aptes à permettre l'accès aux données uniquement pour leur consultation, mais pas pour leur modification.

L'on connaît par ailleurs, du document US 2003/040940 un système d'information médical comprenant un ou une pluralité de serveurs de bases de données reliés en réseau, lesdits serveurs contenant les dossiers médicaux des patients, et des informations médicales diverses. L'accès aux serveurs est basé sur l'utilisation d'un compact disc, de type CD-ROM ou DVD, de préférence au format carte de crédit, servant de carte santé personnelle et faisant office de clé d'accès sécurisée pour accéder et communiquer avec les serveurs, via Internet.

La carte santé peut additionnellement contenir des informations de première urgence, accessibles directement, sans nécessiter de connexion Internet pour accéder à la base de données hébergée par l'un des serveurs.

Selon une des formes de réalisation possibles, tout le dossier médical d'un patient peut être stocké (sous forme cryptée) sur le CD-ROM ou DVD faisant office de carte santé pour être lu ou modifié, en utilisant n'importe quel ordinateur équipé d'un lecteur de CD/DVD apte à lire et écrire.

Pour accéder aux informations stockées sur le CD-ROM ou DVD, un logiciel de cryptage/décryptage, contenu sur ledit CD-ROM ou DVD, est d'abord téléchargé sur l'ordinateur utilisé, puis les données sont décryptées. De même, de nouvelle données sont d'abord cryptées au moyen du même logiciel puis transférées vers le CD-ROM ou DVD au moyen d'un lecteur de CD/DVD apte à lite et écrire.

Le dispositif objet du document US 2003/040940 nécessite par conséquent l'installation d'un logiciel sur l'ordinateur utilisé pour consulter ou modifier les données médicales d'un patient.

Un autre dispositif connu est décrit dans le document US 2002/123909 et se présente sous la forme d'un support, tel un CD-ROM, destiné à recevoir les informations personnelles médicales d'un patient. Ce support comprend tous les logiciels nécessaires à visualiser, modifier et sauvegarder lesdites données.

Toutefois, ce support n'inclut pas d'application permettant la gravure des données directement sur le CD, et il nécessite l'utilisation des logiciels et application de gravure présentes sur l'ordinateur, voir l'installation provisoire d'un logiciel pour la gravure des données modifiées.

Un autre dispositif similaire est divulgué au travers du document US 2003/088439 dans lequel un support portable permet de stocker des données personnelles médicales mais aussi de les visualiser, modifier et sauvegarder. Pour ce faire, un tel support, préférentiellement une disquette 3,5 pouces, comprend une gestionnaire de base de données embarqué qui s'exécute depuis ladite disquette en mémoire temporaire RAM.

Mais un tel dispositif est basé sur un support dont le standard est dépassé et qui n'apporte pas les capacités de stockage requises ou des conditions de sécurisé suffisantes en termes de restriction d'accès auxdites données. De plus, la modification des données peut être opérée partiellement, facilitant les risques de falsification. Enfin, il utilise directement les logiciels d'enregistrement sur disquette, au travers notamment des drivers du lecteur de disquette. Un tel dispositif n'apporte donc pas entière satisfaction.

Contrairement aux dispositifs précités, le dispositif selon l'invention permet non seulement à la fois de consulter et de modifier les données stockées sur le support mobile d'information, en l'occurrence un CD-ROM ou un DVD, mais en outre ce dernier est muni de moyens lui permettant d'être utilise à partir de n'importe quel ordinateur, sans nécessiter l'installation préalable d'un programme, et permettant de restituer cet ordinateur, à la fin de son utilisation dans son état d'origine.

A cet effet, l'invention concerne un dispositif de saisie, de consultation et de traitement de données, notamment médicales, tel que précédemment défini, dans lequel le support mobile comprend des moyens permettant la lecture des données qui y sont enregistrées et l'écriture de nouvelles données, lesdits moyens étant aptes à s'exécuter entièrement à partir dudit support mobile.

Un des avantages d'une telle caractéristique est par exemple défini par le fait qu'elle permet d'optimiser notablement le suivi d'un malade par une équipe composée de plusieurs personnes, appartenant à différents services ou spécialités médicales, et pouvant chacune accéder aux informations réunies sur un même support informatique pouvant être utilisé à partir de n'importe quel ordinateur.

Ainsi, chaque professionnel a la possibilité de consulter et/ou compléter le dossier médical du malade avec les informations dont il relève, à partir de son ordinateur personnel, avant de remettre le support informatique mobile au malade, chargé de le conserver, et pouvant lui-même, s'il le souhaite, accéder aux informations le concernant depuis l'ordinateur de son choix, au moment de son choix.

Bien entendu, si le dispositif selon l'invention a également pour vocation de permettre aux personnes autorisées par le malade de lire son dossier médical, les informations qu'il contient doivent néanmoins être protégées en vue d'empêcher leur accès à toute personne non autorisée par le propriétaire du support informatique.

Ce but est atteint grâce à des moyens aptes à identifier l'utilisateur du support informatique mobile et autoriser ou refuser totalement l'accès aux données qui y sont enregistrées.

D'autre part, de sorte à garantir une sécurité maximale et la pertinence des données enregistrées, l'invention prévoit également que ces mêmes moyens d'identification de l'utilisateur sont aptes à interdire l'écriture de nouvelles données sur le support informatique mobile de sorte à le protéger contre l'écriture de données par toute personne non habilitée, par exemple le propriétaire lui-même, ou une personne qui ne serait pas médecin.

L'invention prévoit également la possibilité, en cas de perte du support informatique mobile, d'invalider ce dernier puis d'en reconstituer un nouveau avec des données identiques à celles qu'il contenait au moment de sa perte.

Cette dernière caractéristique implique l'existence d'une base de données distante hébergée par exemple par une société spécialisée dans ce type d'activités, cette base de données contenant une copie du dossier médical de chaque personne concernée, ainsi que les paramètres nécessaires à la gestion des différents supports informatiques mobiles, tels que les identifiants, les notions de validité, de perte, etc.

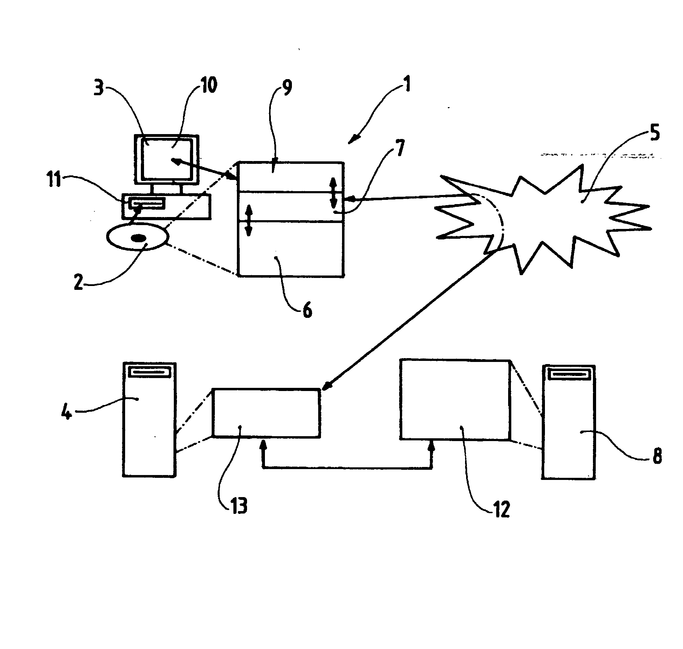

L'invention sera mieux comprise à la lecture de la description qui va suivre, se rapportant à un exemple de réalisation donné à titre indicatif et non limitatif, et dont l'architecture générale est représentée schématiquement sur la figure jointe en annexe.

L'invention a pour objet un dispositif 1 de saisie, de consultation et de traitement de données, notamment médicales, comprenant au moins un support informatique mobile, en l'occurrence un CD-ROM 2, permettant l'enregistrement de données, un lecteur-graveur 3 dudit support mobile 2, tel qu'un ordinateur et éventuellement au moins un support informatique fixe pour l'enregistrement de données, tel qu'un serveur de bases de données 4, le CD-ROM 2 et le serveur de bases de données 4 étant aptes à communiquer entre eux à distance, pour échanger des données, via un réseau de communication 5, de type Internet par exemple, au travers de l'ordinateur 3.

Les premières versions commercialisées du dispositif 1 seront de préférence accompagnées de CD-ROM 2 d'un format de 12 cm, qui non seulement correspondent actuellement au meilleur moyen d'associer une capacité de stockage suffisante et un faible coût, mais sont aussi compatibles avec la plupart des différents types de graveurs qui équipent les ordinateurs des médecins.

Bien entendu, il peut être prévu que le dispositif 1 selon l'invention soit associé à des supports informatiques de type DVD, l'idéal étant le DVD au format « carte de visite » qui allie un faible encombrement et une capacité de stockage suffisante. Ce qui nécessite toutefois que les graveurs de DVD soient proposés de manière standard dans les configurations d'ordinateurs commercialisées.

La principale caractéristique du dispositif 1 est définie par le fait que le CD-ROM 2 comprend tous les moyens nécessaires à sa lecture et à son écriture. C'est-à-dire les informations relatives au dossier médical, sous forme de données cryptées 6, et l'application 7 nécessaire à la fois à la lecture, à l'écriture du dossier médical, et à la communication et à l'envoi des informations vers la base de donnée distante hébergée par le serveur de base de données 4, qui contient une copie de toutes les données saisies sur le CD-ROM 2 ou est reliée à un autre serveur 8 contenant cette même copie.

Avantageusement, lors de l'introduction du CD-ROM 2 dans le lecteur de l'ordinateur 3 de l'utilisateur, l'application 7 est apte à se lancer automatiquement, sans nécessiter d'installation préalable sur ce même ordinateur 3.

Lorsque l'utilisation du CD-ROM 2 est terminée, l'ordinateur 3 de l'utilisateur est restitué dans son état d'origine par l'application 7, dont l'exécution ne requiert qu'une éventuelle écriture de données temporaires.

D'autre part, l'application 7 est portable, de préférence développée en langage "Java" et apte à s'exécuter sur les OS (Operating System) les plus utilisés (Windows xxx, MAC OS), ce qui permet d'utiliser le CD-ROM 2 depuis la plupart des modèles d'ordinateurs 3 dont sont équipés aussi bien les professionnels que les particuliers.

L'application 7 est par ailleurs prévue suffisamment légère pour que la plus grande partie du CD-ROM 2 puisse être consacrée pour le stockage du dossier médical, et non pas pour le stockage de l'application 7.

Le CD-ROM 2 présente de préférence une architecture client/serveur dans laquelle le serveur est défini par un serveur HTTP (HyperText Transfer Protocol) 9 et un "conteneur de servlet", tandis que le client est défini par le navigateur 10 par défaut de l'ordinateur 3, une "servlet" consistant en une application en langage "Java" hébergée sur le serveur et pouvant être exécutée à partir de tout terminal relié à ce serveur.

Lorsque le CD-ROM 2 est introduit dans le lecteur 11 de l'ordinateur 3, l'application 7 démarre sur une fenêtre de choix qui selon le cas lance ou non le serveur HTTP 9 et ouvre le navigateur 10 sur la page d'accueil de l'application 7.

En fait, grâce aux moyens d'identification de l'utilisateur, dont le dispositif 1 est équipé, et qui supposent, par exemple, la saisie d'un identifiant, l'application 7 peut soit autoriser, soit interdire totalement l'accès aux données 6 enregistrées sur le CD-ROM 2, ou encore autoriser ou interdire leur accès pour l'écriture.

Ainsi, un utilisateur, tel qu'un malade, peut être autorisé à accéder aux données 6 pour les consulter uniquement, sans pouvoir les modifier, tandis qu'un autre utilisateur, tel qu'un médecin a la possibilité à la fois de consulter et de modifier ces données 6, par exemple pour en rajouter de nouvelles, pour en supprimer, ou encore les rectifier.

L'application 7 utilise en outre pour son fonctionnement, à travers des "servlets", un module de gravure, permettant la mise à jour du CD-ROM 2 par l'enregistrement des modifications apportées aux données, un module de cryptage et de décryptage des données, un module de construction de pages HTML (Hyper Text Markup Language), et un module permettant une communication avec le serveur de base de données 4, via une transmission cryptée.

Plus précisément, le module de gravure appartenant à l'application 7 comprend deux parties, dont une écrite en langage "Java" contenue dans un ensemble de fichiers "byte code" "Java", et l'autre écrite en langage "C", fournie sous forme de deux librairies.

Le code écrit en "Java" est composé de deux sous-ensembles, à savoir un sous-ensemble capable de construire le fichier "ISO" (+ Jolliet, etc...)" qui va être gravé, et un sous-ensemble utilisant le "Java Native Interface" de "Java", et qui fait appel à des fonctions en langage "C" liées au système d'exploitation.

L'une des deux librairies appartenant à la partie du module de gravage écrite en langage "C", contient les fonctions propres à la gravure (drivers, etc...), et est complètement liée au système d'exploitation, tandis que l'autre librairie contient des fonctions faisant l'interface entre la partie écrite en "Java", et la librairie précédente.

En fait, il y a autant de couples de librairies qu'il y a de systèmes d'exploitations supportés par le module.

Tous les éléments du module de gravure, et de l'application 7 l'utilisant, sont installés sur le CD-ROM 2 ou, le cas échéant, le DVD, ainsi qu'une machine virtuelle "Java" par système d'exploitation.

Pour graver de nouveaux éléments sur le CD-ROM 2, le module de gravure commence par détecter le système d'exploitation sur lequel il est installé, puis il crée en mémoire tous les "objets Java" dont il a besoin pour graver. Il crée ensuite en mémoire le fichier "ISO" qui va être gravé. Puis il charge en mémoire, et en fonction du système d'exploitation détecté, les deux "librairies" en langage "C" nécessaires. Enfin, il appelle dans les "librairies" les fonctions de gravure liées au système d'exploitation, la gravure se faisant par défaut en mode "multi sessions".

Suite à cet enchaînement d'opérations, le CD-Rom 2, sur lequel était installée l'application 7 et le module de gravure, contient les nouveaux éléments.

Une des vocations du serveur de base de données 4 est plus particulièrement d'héberger les sauvegardes des données 6 enregistrées sur les différents CD-ROM 2 mis à disposition des patients.

Ainsi, en cas de perte d'un CD-ROM 2, il suffit d'en reconstituer un neuf, à partir des données enregistrées sur le serveur de base de données 4.

D'ailleurs, chaque CD-ROM 2 comprend également à cet effet des moyens permettant, si nécessaire, de l'invalider.

Le serveur de base de données 4 contient ou est relié à des moyens de traitement des données médicales, tels que notamment une base de données 12 répertoriant des symptômes et des pathologies pouvant servir, dans certains cas, d'assistance médicale au diagnostic.

Ainsi, le serveur de base de données 4 est prévu apte à recevoir, par le biais du réseau de communication 5, des requêtes lancées à partir du CD-ROM 2, et à réaliser ce qui est demandé grâce à une application 13 appropriée, pour notamment permettre l'utilisation de l'assistance médicale au diagnostic.

## Revendications

1. Dispositif de saisie, de consultation et de traitement de données, notamment médicales, comprenant au moins un support informatique mobile (2) pour l'enregistrement de données sous forme d'un CD-ROM ou DVD, un terminal comprenant un lecteur-graveur (3) dudit support mobile (2) et au moins un support informatique fixe (4) pour l'enregistrement de données apte à communiquer à distance avec le support mobile (2) au travers dudit lecteur-graveur (3) de support mobile (2), via un réseau de communication (5), de type Internet, ledit support mobile (2) comprenant des moyens de lecture/écriture ne s'installant pas sur le terminal et y étant aptes à s'exécuter entièrement à partir dudit support mobile et permettant à la fois la lecture des données qui y sont enregistrées et l'écriture sur ledit support mobile de nouvelles données, **caractérisé par le fait que** lesdits moyens de lecture/écriture sont définis par une application comprenant un module de gravure, ledit module de gravure est constitué d'une partie écrite en langage "Java" et d'une autre partie écrite en langage "C", la partie écrite en langage "Java" étant composée d'un sous-ensemble capable de construire le fichier "Iso" destiné à être gravé, et d'un sous-ensemble utilisant le "Java Native Interface", tandis que la partie écrite en langage "C" est prévue sous forme de deux librairies, l'une d'entre elles contenant des fonctions faisant l'interface entre la partie écrite en langage "Java" et la librairie contant les fonctions propres à la gravure.

2. Dispositif selon la revendication 1, **caractérisé en ce qu'**il comprend des moyens aptes à identifier l'utilisateur du support informatique mobile (2) pour autoriser ou interdire l'accès aux données qui s'y trouvent enregistrées pour leur lecture, et autoriser ou interdire l'écriture de nouvelles données.

3. Dispositif selon l'une quelconque des revendications précédentes, **caractérisé en ce qu'**il comprend des moyens aptes, le cas échéant, à invalider le support informatique mobile (2).

4. Dispositif selon l'une quelconque des revendications précédentes, **caractérisé en ce que** lesdits moyens de lecture et d'écriture de données sur le support mobile comprennent des moyens de cryptage et de décryptage desdites données.

5. Dispositif selon l'une quelconque des revendications précédentes, **caractérisé en ce que** lesdits moyens de lecture et d'écriture de données sur le support mobile comprennent des moyens de construction de pages HTML.

6. Dispositif selon l'une quelconque des revendications précédentes, **caractérisé en ce que** lesdits moyens de lecture et d'écriture de données sur le support mobile (2) comprennent des moyens aptes à permettre une communication entre les données du support informatique mobile et les données du support informatique fixe (4).

7. Dispositif selon l'une quelconque des revendications précédentes, **caractérisé en ce que** ledit support fixe d'enregistrement de données est défini par un serveur informatique (4) contenant une base de données.

8. Dispositif selon l'une quelconque des revendications précédentes, **caractérisé en ce que** ledit lecteur-graveur dudit support mobile est défini pour un ordinateur.

## Claims

1. Device for inputting, consulting and processing data, namely medical data, comprising at least one mobile data-processing carrier (2) for recording data in the form of a CD-ROM or DVD, a terminal comprising a reader-writer (3) for said mobile carrier (2) and at least one fixed data-processing carrier (4) for recording data capable of communicating remotely with the mobile carrier (2) through said reader-writer (3) of the mobile carrier (2), via a communication network (5), such as Internet, said mobile carrier (2) comprising reading/writing means that are not installed on the terminal and are capable of being fully executed thereon from said mobile carrier and permitting both to read data recorded thereon and to write new data on said mobile carrier, wherein said reading/writing means are defined by an application comprising a writer module, said writer module is formed by a portion written in "Java" language and a portion written in "C" language, the portion written in "Java" language being comprised of a subset capable of constructing the "lso" file aimed at being written, and a subset using the "Java Native Interface", while the portion written in "C" language is designed in the form of two libraries, one of them containing functions constituting the interface between the portion written in "Java" language and the library containing the functions related to the writing.

2. Device according to claim 1, wherein it comprises means capable of identifying the user of the mobile data-processing carrier (2) in order to authorise or prevent the access to the data recorded thereon for their reading, and to authorise or prevent the writing of new data.

3. Device according to any of the preceding claims, wherein it comprises means capable, should the case arise, of invalidating the mobile data-processing carrier (2).

4. Device according to any of the preceding claims, wherein said means for reading and writing data on the mobile carrier comprise means for encrypting and decrypting said data.

5. Device according to any of the preceding claims, wherein said means for reading and writing data on the mobile carrier comprise means for constructing HTML pages.

6. Device according to any of the preceding claims, wherein said means for reading and writing data on the mobile carrier (2) comprise means capable of permitting a communication between the data of the mobile data-processing carrier and the data of the fixed data-processing carrier (4).

7. Device according to any of the preceding claims, wherein said fixed data-recording carrier is defined by a data-processing server (4) containing a database.

8. Device according to any of the preceding claims, wherein said reader-writer of said mobile carrier is defined for a computer.

## Patentansprüche

1. Vorrichtung zum Eingeben, Abfragen und Verarbeiten von Daten, nämlich medizinischen Daten, umfassend wenigstens einen mobilen Datenverarbeitungsträger (2) zum Aufnehmen von Daten in der Form eines CD-ROM oder DVD, ein Endgerät, das eine Lese-Schreibeinrichtung (3) für den besagten mobilen Träger (2) umfaßt, und wenigstens einen festen Datenverarbeitungsträger (4) zum Aufnehmen von Daten, der geeignet ist, über die besagte Lese-Schreibeinrichtung (3) des mobilen Trägers (2) mit dem mobilen Träger (2) fernzukommunizieren, über ein Kommunikationsnetzwerk (5), der Art Internet, wobei der mobile Träger (2) Lese/Schreibmittel umfaßt, die nicht auf dem Endgerät installiert werden und geeignet sind, ab dem besagten mobilen Träger darauf völlig ausgeführt zu werden, und die es erlauben, sowohl darauf aufgenomme Daten zu lesen als auch neue Daten auf den genannten mobilen Träger zu schreiben, **dadurch gekennzeichnet, dass** die besagten Lese/Schreibmittel durch eine Anwendung definiert sind, die ein Schreibmodul umfaßt, das Schreibmodul durch einen Teil, der in "Java"-Sprache geschrieben ist und einen Teil, der in "C"-Sprache gebrieben ist, gebildet ist, wobei der in "Java"-Sprache geschriebene Teil aus einer Subeinheit, die geeignet ist, die "lso"-Datei zu bilden, die dazu bestimmt ist, geschrieben zu werden, und einer Subeinheit, die die "Java Native Interface" verwendet, besteht, während der in "C"-Sprache geschriebene Teil in der Form von zwei Bibliotheken vorgesehen ist, wobei eine davon Funktionen beinhält, die die Schnittstelle zwischen dem in "Java"-Sprache geschriebenen Teil und der Bibliothek, die die dem Schreiben eignen Funktionen beinhält, bilden.

2. Vorrichtung nach Anspruch 1, **dadurch gekennzeichnet, dass** es Mittel umfaßt, die geeignet sind, den Gebraucher des mobilen Datenverarbeitungsträgers (2) zu identifizieren, un den Zugang zu den darauf aufgenommenen Daten, um sie zu lesen, zu erlauben oder zu verbieten, und das Schreiben von neuen Daten zu erlauben oder zu verbieten.

3. Vorrichtung nach irgendeinem der vorgehenden Ansprüche, **dadurch gekennzeichnet, dass** es Mittel umfaßt, die geeignet sind, gegebenenfalls den mobilen Datenverarbeitungsträger (2) ungültig zu machen.

4. Vorrichtung nach irgendeinem der vorgehenden Ansprüche, **dadurch gekennzeichnet, dass** die besagten Mittel, um Daten auf dem mobilen Träger zu lesen und zu schreiben, Mittel zum Ver- und Entschlüsseln der besagten Daten umfaßt.

5. Vorrichtung nach irgendeinem der vorgehenden Ansprüche, **dadurch gekennzeichnet, dass** die besagten Mittel, um Daten auf dem mobilen Träger zu lesen und zu schreiben, Mittel zum Konstruieren von HTML-Seiten umfaßt.

6. Vorrichtung nach irgendeinem der vorgehenden Ansprüche, **dadurch gekennzeichnet, dass** die besagten Mittel, um Daten auf dem mobilen Träger (2) zu lesen und zu schreiben, Mittel umfassen, die geeignet sind, eine Kommunikation zwischen den Daten des mobilen Datenverarbeitungsträgers und den Daten des festen Datenverarbeitungsträgers (4) zu erlauben.

7. Vorrichtung nach irgendeinem der vorgehenden Ansprüche, **dadurch gekennzeichnet, dass** der besagte feste Datenaufnahmeträger durch einen Datenverarbeitungsserver (4), der eine Datenbank beinhält, definiert ist.

8. Vorrichtung nach irgendeinem der vorgehenden Ansprüche, **dadurch gekennzeichnet, dass** das besagte Lese-Schreibgerät des besagten mobilen Trägers für einen Computer definiert ist.
